# EUROPEAN PATENT SPECIFICATION

(11) **EP 4 297 822 B1**
(45) Date of publication and mention of the grant of the patent: **24.12.2025**
(21) Application number: 21724059.7
(22) Date of filing: 26.02.2021
(51) Int. Cl.: A61M 5/145, A61M 5/155, A61M 25/10, A61M 39/10

(54) **TWO-STEP INFLATOR WITH EXCESS PROTECTION**
ZWEISTUFIGE AUFBLASVORRICHTUNG MIT ÜBERSPANNUNGSSCHUTZ
GONFLEUR EN DEUX ÉTAPES AVEC PROTECTION CONTRE LES DÉPASSEMENTS

(43) Date of publication of application: 03.01.2024
(73) Proprietor: American Heart of Poland S.A., 43-450 Ustron (PL); Centrum Materialow Polimerowych i Weglowych Polskiej Akademii Nauk, 41-819 Zabrze (PL); Innovations for Heart and Vessels Spolka z Ograniczona Odpowiedzialnoscia, 40-568 Katowice (PL); BALTON Spolka z ograniczona odpowiedzialnoscia, 00-496 Warszawa (PL); Wojskowa Akademia Techniczna, 00-908 Warszawa (PL); Slaskie Centrum Chorob Serca W Zabrzu, 41-800 Zabrze (PL); Fundacja Rozwoju Kardiochirurgii Im. Prof. Zbigniewa Religi, 41-800 Zabrze (PL); Politechnika Slaska, 44-100 Gliwice (PL)
(72) Inventor: BUSZMAN, Piotr, 40-761 Katowice (PL); WOLANSKI, Wojciech, 44-144 Zernica (PL); JOSZKO, Kamil, 42-609 Tarnowskie Góry (PL); GZIK, Marek, 44-100 Gliwice (PL)
(74) Representative: JWP Patent & Trademark Attorneys
(86) International application number: PCT/PL2021/050012
(87) International publication number: WO 2022/182256

(56) References cited:
- WO-A1-97/44077
- US-A- 4 723 938
- US-A- 4 758 223
- US-A- 4 929 238
- US-A1- 2016 279 395
- US-A1- 2018 036 520
- US-A1- 2019 217 068

## Description

The subject matter of the present teaching is a two-step inflator with excess protection, especially for angioplasty procedures using biodegradable stents.

Application US2020100798A1 discloses a device, system and method for inflation. The inflation device includes a housing, a compressed gas canister disposed therein, a syringe disposed within the housing, a plunger slidably disposed in the syringe having an unloaded position and a loaded position relative to the syringe, a puncture mechanism disposed within in the housing and configured to open a fluid path between the compressed gas canister and the syringe, and a positive pressure mechanism disposed within the housing and operably coupled to the syringe. Engagement of the puncture mechanism puts the syringe into fluid communication with the compressed gas canister such that gas contained therein loads into the syringe and the plunger moves from the unloaded position and the loaded position. The positive pressure mechanism is configured to maintain the gas within the syringe at a positive pressure relative to ambient pressure in order to prevent air from entering the syringe after loading.

Application US2013261601A1 discloses an inflation-deflation device that inflates a balloon with controlled pressure. Moving a large plunger provides incremental pressure increases within a syringe. A releasable latch moves a second plunger, positioned within a syringe formed by an inner columnar opening in the large plunger, between an occlusion position and a perfusion position. A three-position switch has a release position to allow the large plunger to move freely, an adjust position to provide the incremental pressure increases, and a lock position to lock the location of the plunger. Pressures at which various balloons achieve or nearly achieve a substantially circular cross-section may be extrapolated by inflating balloons with pressures equal and/or greater than the first occlusion pressure. Balloons may be pre-inflated up to or below the first occlusion pressure to achieve a substantially circular cross-section, prior to increasing the balloon to an occlusion pressure.

Prior art inflators are used for angioplasty procedures with metal stents that require significantly higher operating pressures and are less sensitive to deviations from nominal pressure. For angioplasty procedures using biodegradable stents, applying precise pressure and slowly reaching the nominal value are required. Such conditions being ensured guarantee proper stent implantation and its structural integrity and also result in no damage to the stent and the intended effects being achieved.

It is an object of the present invention to provide a device as set out in the appended claims, for precise pressure application during angioplasty procedures using biodegradable stents.

The two-step inflator with excess protection according to the present teaching comprises a housing and a cylinder secured therein, with an outlet terminating the cylinder in the lower part, with a plunger slidably mounted inside the cylinder and attached to an inner screw, the screw terminated with a handle, wherein the thread of the inner screw engages the inner thread of an outer screw and the outer screw is connected to the handle, with the inflator also comprising a pressure gauge, wherein the outer screw is mounted in the lining of a release brake, the lining connected to a brake release system, while connectors are movably mounted at the outer edges of the lower handle and the connectors connect in the lower position to the housing and prevent the outer screw from moving relative to the housing, and the connectors connect in the upper position to the upper handle of the inner screw and lock the outer screw and the inner screw relative to each other but enable the outer screw and the inner screw to simultaneously move relative to the housing, and further, in the lower part of the cylinder, the inflator comprises a distributor, with a pressure gauge and an adjustable excess valve attached to the distributor.

Preferably, the connectors have semicircular, opposite recesses at the ends of side edges.

Preferably, there are hollows in the upper corners of the housing, with pins mounted in the hollows.

Also preferably, pins are mounted at the edges of the upper handle.

Also preferably, the housing is a two-piece housing.

The two-step inflator with excess protection according to the present teaching utilizes a pressure application mechanism in the form of a two-piece screw piston rod. It enables a two-step pressure application, and the adjustable excess valve attached to the inflator protects the system against exceeding the set value of pressure, and in the case of higher pressure values, redirects the excess liquid in the system to the excess container, thus reducing the pressure.

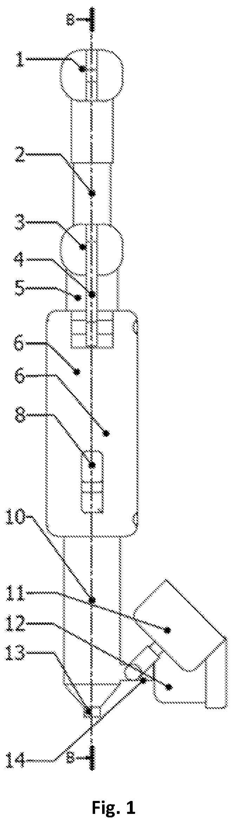

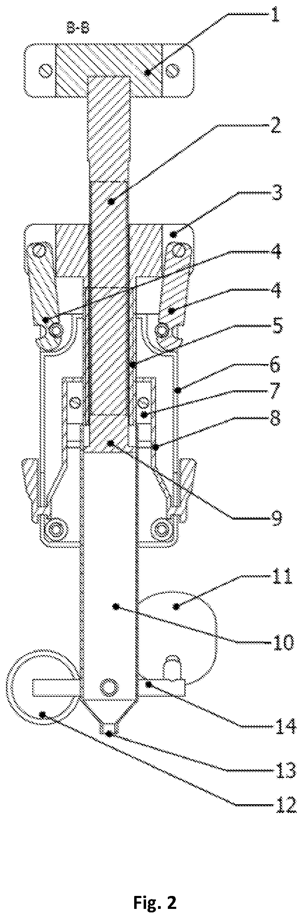

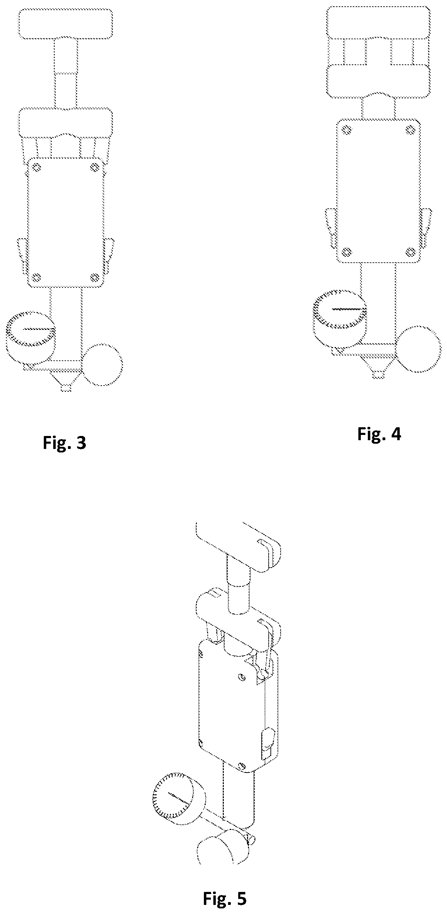

The subject matter of the present teaching is shown in the embodiment in the drawing, wherein Fig. 1 shows the two-step inflator with excess protection in a side view, Fig. 2 shows a cross-section of the two-step inflator along B-B line from Fig. 1, Fig. 3 shows the two-step inflator in the first step of operation in a top view, Fig. 4 shows the two-step inflator in the second step of operation in a top view, Fig. 5 shows the two-step inflator in the first step of operation in a perspective view.

The two-step inflator in an exemplary embodiment comprises a housing 6 and a cylinder 10 secured therein, with an outlet 13 terminating the cylinder in the lower part, with a plunger 9 slidably mounted inside the cylinder and attached to an inner screw 2, the screw terminated with an upper handle 1, and the thread of the inner screw 2 engages the inner thread of an outer screw 5, wherein the outer screw 5 is connected to a lower handle 3. The outer screw 5 has a coarse thread on its outer surface and a pass-through opening inside, which has an inner fine thread over the entire length of its surface. The inner screw 2 has a fine thread on its outer surface, the fine thread having the same pitch as the inner thread of the outer screw 5. The upper handle 1 and the lower handle 3 make it possible to manually apply pressure by turning the screws and to act on the plunger 9 through said screws, where the plunger, by shifting in the cylinder 10, exerts pressure on the liquid contained in the cylinder. Connectors 4 are movably mounted at the outer edges of the lower handle 3 and, in the lower position, the connectors connect to the housing 6 and prevent the outer screw 5 from moving relative to the housing 6. The connection is made by the semicircular recesses in the connectors 4 engaging the pins mounted in the hollows of the housing 6. In contrast, in the upper position, the connectors 4 connect to the upper handle 1 of the inner screw 2. The connection is made by the semicircular recesses in the connectors 4, these connectors located opposite the recesses for the lower position, engaging the pins mounted at the edges of the upper handle 1. The outer screw 5 and the inner screw 2 become locked relative to each other but the outer screw 5 and the inner screw 2 are enabled to simultaneously move relative to the housing 6. The use of the lock acting on the double screw makes it necessary to apply pressure in steps. In the lower part of the cylinder 10 there is an outlet 13 and an installation opening, to which a distributor 14 is attached. A pressure gauge 11 and an adjustable excess valve 12 are connected to the distributor. For emergency situations, a quick backward shift of the screws 2, 5 relative to cylinder 10 is possible with the use of the brake system. The outer screw 5 is mounted in the lining of the release brake 7, the lining connected to the brake release system 8, the clamps of which are disposed on the sides of the housing 6. Pressing the clamps lifts the linings of the release brake 7 and enables quick backward shift to immediately reduce the pressure of the liquid in the cylinder 10.

The operation of the two-step inflator consists of two steps. In the first step, with the connectors 4 in the lower position, by shifting the inner screw 2, it is possible to precisely apply pressure to the value of about 4 atmospheres (approx. 400 kPa). The use of a fine thread ensures low speed of the shift and extended time to reach the limit value of the liquid pressure. With the set value reached for pressure, it is impossible to increase it further without changing the position of the connectors 4 and adjusting the adjustable excess valve 12. Moving the connectors 4 from the lower to the upper position makes it possible to further apply pressure through the unlocked outer screw 5 with the coarse thread. In this step, the pressure increases proportionally to the shift of said screw until it reaches the set value. In the second step, higher pressure values of about 15 atmospheres can be reached. The adjustable excess valve 12 connected to the inflator enables limit pressure ranges to be planned in each step of the inflator operation, the values of which may not be exceeded. This makes it possible to determine pressure ranges in reaching the desired value according to a planned schedule that also includes time intervals. The adjustable excess valve 12 further protects the biodegradable stent against overload. It reduces excess pressure by reducing the liquid in the system, redirecting it to the excess tank 15 (not shown).

### List of reference signs:

1. Upper handle
2. Inner screw
3. Lower handle
4. Connector
5. Outer screw
6. Housing
7. Linings of the release brake
8. Brake release system
9. Plunger
10. Cylinder
11. Pressure gauge
12. Adjustable excess valve
13. Outlet
14. Distributor
15. Excess tank

The present invention is set out in the claims that follow.

## Claims

1. Two-step inflator comprising a housing (6) and a cylinder (10) secured therein and configured to contain a liquid, an outle (13) arranged in the lower part of the cylinder (10) and terminating said cylinder (10), a plunger (9) slidably mounted inside the cylinder (10), wherein the lower part of the cylinder further comprises a distributor (14), a pressure gauge (11) and an adjustable excess valve (12) attached to the distributor (14), the two-step inflator further comprising a handle comprising a upper handle (1) and a lower handle (3), an inner screw (2) having an outer thread, an outer screw (5) having an inner thread, wherein the inner screw (2) terminates with the upper handle (1) and the outer screw (5) terminates with the lower handle (3), wherein the outer thread of the inner screw (2) is configured to engage the inner thread of the outer screw (5), wherein the upper and lower handles (1, 3) are configured to act on the plunger (9) via the inner and outer screws, the two-step inflator further comprising a brake release system (8) comprising clamps disposed on the sides of the housing (6), and a release brake (7) comprising a lining, wherein the outer screw (5) is mounted in the lining of the release brake (7), wherein the two-step inflato further comprises connectors (4) mounted at the outer edges of the lower handle (3) wherein the connectors (4) are configured to be moved between an upper position in which the connectors (4) connect to the upper handle (1) and a lower position in which the connectors (4) connect to the housing (6), wherein, in the lower position, the connectors (4) prevent the outer screw (5) from moving relative to the housing (6), and wherein, in the upper position, the connectors (4) are configured to lock the outer screw (5) and the inner screw (2) relative to each other such that, in the upper position, the outer screw (5) and the inner screw are configured to simultaneously move relative to the housing (6) upon actuation of the upper handle (1), and wherein the clamps are configured to be pressed and wherein, upon pressing, the clamps are configured to lift the lining of the release brake (7) thereby enabling quick backward shift of the inner screw and outer screws (2, 5) within the cylinder to immediately reduce the pressure of the liquid in the cylinder (10).

2. The two-step inflator according to claim 1, wherein the connectors (4) have semicircular, opposite recesses at the ends of side edges.

3. The two-step inflator according to claim 1, wherein in the upper corners of the housing (6) there are hollows in which pins are mounted.

4. The two-step inflator according to claim 1, wherein pins are mounted at the edges of the upper handle (1).

5. The two-step inflator according to claim 1, wherein the housing (6) is a two-piece housing.

6. The two-step inflator according to claim 1, wherein the outer screw (5) comprises a coarse thread on its outer surface.

7. The two-step inflator according to claim 6 wherein the outer screw (5) comprises a pass through opening inside, which has an inner fine thread over the entire length of its surface.

8. The two-step inflator according to claim 7, wherein the inner screw comprises a fine thread on its outer surface, the fine thread having the same pitch as the inner thread of the outer screw.

## Patentansprüche

1. Zweistufiger Inflator, bestehend aus einem Gehäuse (6) und einem darin befestigten Zylinder (10), der zur Aufnahme einer Flüssigkeit ausgelegt ist, einem im unteren Teil des Zylinders (10) angeordneten und diesen Zylinder (10) abschließenden Auslass (13), einen Kolben (9), der verschiebbar im Zylinder (10) angebracht ist, wobei der untere Teil des Zylinders ferner einen Verteiler (14), ein Manometer (11) und ein einstellbares Überdruckventil (12) umfasst, das am Verteiler (14) angebracht ist, wobei der zweistufige Inflator ferner einen Griff umfasst, der einen oberen Griff (1) und einen unteren Griff (3) umfasst, eine Innenschraube (2) mit einem Außengewinde, eine Außenschraube (5) mit einem Innengewinde, wobei die Innenschraube (2) mit dem oberen Griff (1) endet und die Außenschraube (5) mit dem unteren Griff (3) endet, wobei das Außengewinde der Innenschraube (2) so konfiguriert ist, dass es in das Innengewinde der Außenschraube (5) in Eingriff kommt, wobei der obere und der untere Griff (1, 3) so konfiguriert sind, dass sie über die innere und die äußere Schraube auf den Kolben (9) einwirken, wobei der zweistufige Inflator ferner ein Bremsfreigabesystem (8) umfasst, das an den Seiten des Gehäuses (6) angeordnete Klemmen umfasst, sowie eine Auslösebremse (7) mit einer Auskleidung, wobei die Außenschraube (5) in der Auskleidung der Auslösebremse (7) montiert ist, wobei der zweistufige Inflator ferner Verbindungsstücke (4) umfasst, die an den Außenkanten des unteren Griffs (3) montiert sind, wobei die Verbindungsstücke (4) so konfiguriert sind, dass sie zwischen einer oberen Position, in der die Verbindungsstücke (4) mit dem oberen Griff (1) verbunden sind, und einer unteren Position, in der die Verbindungsstücke (4) mit dem Gehäuse (6) verbunden sind, beweglich sind, wobei in der unteren Position die Verbindungsstücke (4) verhindern, dass sich die Außenschraube (5) relativ zum Gehäuse (6) bewegt, und wobei in der oberen Position die Verbindungsstücke (4) so konfiguriert sind, dass sie die Außenschraube (5) und die Innenschraube (2) relativ zueinander so arretieren, dass in der oberen Position die Außenschraube (5) und die Innenschraube bei Betätigung des oberen Griffs (1) relativ zum Gehäuse (6) gleichzeitig bewegbar sind, und wobei die Klemmen so konfiguriert sind, dass sie gedrückt werden können, und wobei die Klemmen beim Drücken so konfiguriert sind, dass sie die Auskleidung der Auslösebremse (7) anheben, wodurch eine schnelle Rückwärtsverschiebung der inneren Schraube und der äußeren Schrauben (2, 5) innerhalb des Zylinders ermöglicht wird, um den Druck der Flüssigkeit im Zylinder (10) zu reduzieren.

2. Zweistufiger Inflator gemäß Anspruch 1, wobei die Verbindungsstücke (4) an den Enden der Seitenkanten halbkreisförmige, gegenüberliegende Aussparungen aufweisen.

3. Zweistufiger Inflator gemäß Anspruch 1, wobei an den oberen Ecken des Gehäuses (6) Vertiefungen vorhanden sind, in denen Stifte angebracht sind.

4. Zweistufiger Inflator nach Anspruch 1, wobei Stifte an den Kanten des oberen Griffs (1) angebracht sind.

5. Zweistufiger Inflator nach Anspruch 1, wobei das Gehäuse (6) ein zweiteiliges Gehäuse ist.

6. Zweistufiger Inflator nach Anspruch 1, wobei die Außenschraube (5) an ihrer Außenfläche ein Grobgewinde aufweist.

7. Zweistufiger Inflator nach Anspruch 6, wobei die Außenschraube (5) eine Durchgangsöffnung im Inneren aufweist, die über ihre gesamte Länge ein inneres Feingewinde aufweist.

8. Zweistufiger Inflator nach Anspruch 7, wobei die Innenschraube ein Feingewinde an ihrer Außenfläche aufweist, wobei das Feingewinde die gleiche Steigung wie das Innengewinde der Außenschraube aufweist.

## Revendications

1. Gonfleur à deux étages comprenant un boîtier (6) et un cylindre (10) fixé à l'intérieur et configuré pour contenir un liquide, une sortie (13) disposée dans la partie inférieure du cylindre (10) et terminant ledit cylindre (10), un piston (9) monté de manière coulissante à l'intérieur du cylindre (10), dans lequel la partie inférieure du cylindre comprend en outre un distributeur (14), un manomètre (11) et une soupape de décharge réglable (12) fixée au distributeur (14), dans lequel le gonfleur en deux étapes comprend en outre une poignée composée d'une poignée supérieure (1) et d'une poignée inférieure (3), dans lequel une vis intérieure (2) ayant un filetage extérieur, dans lequel la vis extérieure (5) a un filetage intérieur, dans lequel la vis intérieure (2) se termine par la poignée supérieure (1) et la vis extérieure (5) se termine par la poignée inférieure (3), dans lequel le filetage extérieur de la vis intérieure (2) est configuré pour s'engager dans le filetage intérieur de la vis extérieure (5), dans lequel les poignées supérieure et inférieure (1,3) sont configurées pour agir sur le plongeur (9) par l'intermédiaire des vis intérieure et extérieure, dans lequel le gonfleur en deux étapes comprend en outre un système de libération du frein (8) comprend des pinces disposées sur les côtés du boîtier (6), et dans lequel un frein de libération (7) comprend une garniture, dans lequel la vis extérieure (5) est montée dans la garniture du frein à main (7), dans lequel le gonflement en deux étapes comprend en outre des connecteurs (4) montés sur les bords extérieurs de la poignée inférieure (3), dans lequel les connecteurs (4) sont configurés pour être déplacés entre une position supérieure dans laquelle les connecteurs (4) se connectent à la poignée supérieure (1) et une position inférieure dans laquelle les connecteurs (4) se connectent au boîtier (6), dans lequel, en position basse, les connecteurs (4) empêchent la vis extérieure (5) de se déplacer par rapport au boîtier (6), et dans lequel, dans la position supérieure, les connecteurs (4) sont configurés pour verrouiller la vis extérieure (5) et la vis intérieure (2) l'une par rapport à l'autre de sorte que, dans la position supérieure, la vis extérieure (5) et la vis intérieure sont configurées pour se déplacer simultanément par rapport au boîtier (6) lors de l'actionnement de la poignée supérieure (1), et dans lequel les pinces sont configurées pour être pressées et dans lequel, lors de la pression, les pinces sont configurées pour soulever la garniture du frein de déblocage (7) permettant ainsi un déplacement rapide vers l'arrière de la vis intérieure et des vis extérieures (2, 5) à l'intérieur du cylindre pour réduire immédiatement la pression du liquide dans le cylindre (10).

2. Gonfleur en deux étapes selon la revendication 1, dans lequel les connecteurs (4) ont des évidements semi-circulaires opposés aux extrémités des bords latéraux.

3. Gonfleur en deux étapes selon la revendication 1, dans lequel les coins supérieurs du boîtier (6) présentent des creux dans lesquels des goupilles sont montées.

4. Gonfleur en deux étapes selon la revendication 1, dans lequel les goupilles sont montées sur les bords de la poignée supérieure (1).

5. Gonfleur en deux étapes selon la revendication 1, dans lequel le boîtier (6) est un boîtier en deux parties.

6. Gonfleur en deux étapes selon la revendication 1, dans lequel la vis extérieure (5) comprend un filetage grossier sur sa surface extérieure.

7. Gonfleur en deux étapes selon la revendication 6, dans lequel la vis extérieure (5) comprend une ouverture de passage à l'intérieur, qui a un filetage intérieur fin sur toute la longueur de sa surface.

8. Gonfleur en deux étapes selon la revendication 7, dans lequel la vis intérieure comprend un filetage fin sur sa surface extérieure, le filetage fin ayant le même pas que le filetage intérieur de la vis extérieure.
